# EUROPEAN PATENT SPECIFICATION

(11) **EP 4 260 802 B1**
(45) Date of publication and mention of the grant of the patent: **04.11.2026**
(21) Application number: 23194372.1
(22) Date of filing: 01.09.2015
(51) Int. Cl.: A61B 5/00, A61B 5/024

(54) **A HEART RATE SENSING WEARABLE DEVICE**
TRAGBARE HERZFREQUENZMESSVORRICHTUNG
DISPOSITIF PORTABLE DE DÉTECTION DE FRÉQUENCE CARDIAQUE

(43) Date of publication of application: 18.10.2023
(62) Document number(s) of the earlier application(s) in accordance with Art. 76 EPC: 15306343.3 / 3 138 478
(73) Proprietor: Essilor International, 94220 Charenton-Le-Pont (FR)
(72) Inventor: ROUSSEAU, Denis, 94220 Charenton-le-Pont (FR); PERRIER, Eric, 94220 Charenton-le-Pont (FR)
(74) Representative: Cabinet Novitech

(56) References cited:
- US-A1- 2014 121 982
- US-A1- 2014 159 862
- US-A1- 2015 005 653
- US-A1- 2015 045 650
- US-A1- 2015 051 468

## Description

### FIELD OF THE INVENTION

The invention relates to a heart rate sensing module configured to be fixed to a wearable device intended to be worn by a user. The invention further relates to a heart rate determining component for determining the heart rate of a wearer of a heart rate sensing module according to the invention and to a wearable device comprising a heart rate sensing module according to the invention.

### BACKGROUND OF THE INVENTION

Sensing heart rate of a user of a wearable device may provide useful information concerning the user, in particular concerning the health condition of the user. Most of the existing heart rate sensing devices requires a contact with the skin of the user. When using a wearable device on an everyday bases it may be complex ensuring permanent contact between the heart rate sensing device and the skin of the user.

A wearable heart rate sensing device is known from US 2014/121982.

Thus there is a need for heart rate sensing module that would not require contact with the user's skin.

One object of the present invention is to provide a monitoring component allowing monitoring the heart rate of a user of a wearable device.

### SUMMARY OF THE INVENTION

To this end, the invention proposes a heart rate sensing module configured to be fixed on a wearable device intended to be worn by a user, the module comprising:
- at least one contactless sensor adapted to sense at least one parameter indicative of the distance between at least a reference point of the skin of the user and a reference point of the module when the module is fixed to the wearable device when worn by the user in such a manner that the contactless sensor is distant from the skin of the user,
- at least one communication entity adapted to communicate data indicative of the at least one parameter sensed by the at least one sensor to a heart rate determining component.

The sensing module according to the invention allows monitoring the heart rate of the user without contact between the sensor and the skin of the user.

Advantageously, such sensing module may be placed, for example on a spectacle frame, in a greater diversity of positions than the sensing devices requiring contact with the skin.

According to further embodiments which can be considered alone or in combination:
- the heart rate sensing module is configured to be fixed on a spectacle frame; and/or
- the heart rate sensing module is configured so that when fixed on the wearable device and said wearable device worn by the user, the contactless sensor faces an artery or vein of the user; and/or
- the contactless sensor is a magnetic sensor; and/or
- the at least one parameter relates to the of magnetic field measured by the magnetic sensor.

The disclosure also relates to a heart rate determining component for determining the heart rate of a wearer of a wearable sensing device according to the invention, the heart rate determining component comprising:
- a communication unit configured to receive data indicative of the at least one parameter sensed by the at least one sensor of a heart rate sensing module according to the invention, and
- a heart rate unit comprising at least
- a memory configured to store computer executable instructions; and
- a processor for executing the computer executable instructions,
wherein the computer executable instructions comprises instructions for determining heart rate data indicative of the heart rate of the user based on the received data.

According to further embodiments which can be considered alone or in combination:
- the computer executable instructions further comprises instructions for determining heart rate data based on the variation over time of the distance between the at least a reference point of the skin of the user and a reference point of the module when the module is fixed to the wearable device worn by the user; and/or
- further comprising an information generating unit configured to generate information based on the heart rate data; and/or
- the information comprises recommendation data including a change of activity recommendation and/or an alert indicative of the user's heart state and/or an access to a service offer; and/or
- the communication unit is configured to send heart rate related data to a distant entity, for example a wearable computer device and/or a personal computer device, associated to the user of the heart rate determining component.

A device in accordance with the invention is defined in claim 1.

The wearable device may be a head mounted device comprising a frame to be mounted on the head of the wearer and the heart rate sensing module is fixed to the frame.

Unless specifically stated otherwise, as apparent from the following discussions, it is appreciated that throughout the specification discussions utilizing terms such as "computing", "calculating", or the like, refer to the action and/or processes of a computer or computing system, or similar electronic computing device, that manipulate and/or transform data represented as physical, such as electronic, quantities within the computing system's registers and/or memories into other data similarly represented as physical quantities within the computing system's memories, registers or other such information storage, transmission or display devices.

Embodiments of the present invention may include apparatuses for performing the operations herein. This apparatus may be specially constructed for the desired purposes, or it may comprise a general purpose computer or Digital Signal Processor ("DSP") or FPGA selectively activated or reconfigured by a computer program or computer executable instructions stored in the computer. Such a computer program or computer executable instructions may be stored in a computer readable storage medium, such as, but is not limited to, any type of disk including floppy disks, optical disks, CD-ROMs, magnetic-optical disks, read-only memories (ROMs), random access memories (RAMs), ferro magnetic RAM, electrically programmable read-only memories (EPROMs), electrically erasable and programmable read only memories (EEPROMs), magnetic or optical cards, or any other type of media suitable for storing electronic instructions, and capable of being coupled to a computer system bus.

The processes and displays presented herein are not inherently related to any particular computer or other apparatus. Various general purpose systems may be used with programs in accordance with the teachings herein, or it may prove convenient to construct a more specialized apparatus to perform the desired method.

The desired structure for a variety of these systems will appear from the description below. In addition, embodiments of the present invention are not described with reference to any particular programming language. It will be appreciated that a variety of programming languages may be used to implement the teachings of the inventions as described herein.

### BRIEF DESCRIPTION OF THE DRAWINGS

Embodiments of the invention will now be described, by way of example only, and with reference to the following drawings in which:
- Figure 1 is a schematic representation of a heart rate sensing module according to the invention,
- Figure 2 is a schematic representation of a heart rate determining component according to the invention,
- Figure 3 is a schematic representation of an head mounted device according to an embodiment of the invention;
- Figure 4 represents a networked data-processing device according to the invention; and
- Figure 5 is a schematic representation of a head mounted device according to a further embodiment of the invention.

### DETAILED DESCRIPTION OF PREFERRED EMBODIMENTS

Elements in the figures are illustrated for simplicity and clarity and have not necessarily been drawn to scale. For example, the dimensions of some of the elements in the figure may be exaggerated relative to other elements to help improve the understanding of the embodiments of the present invention.

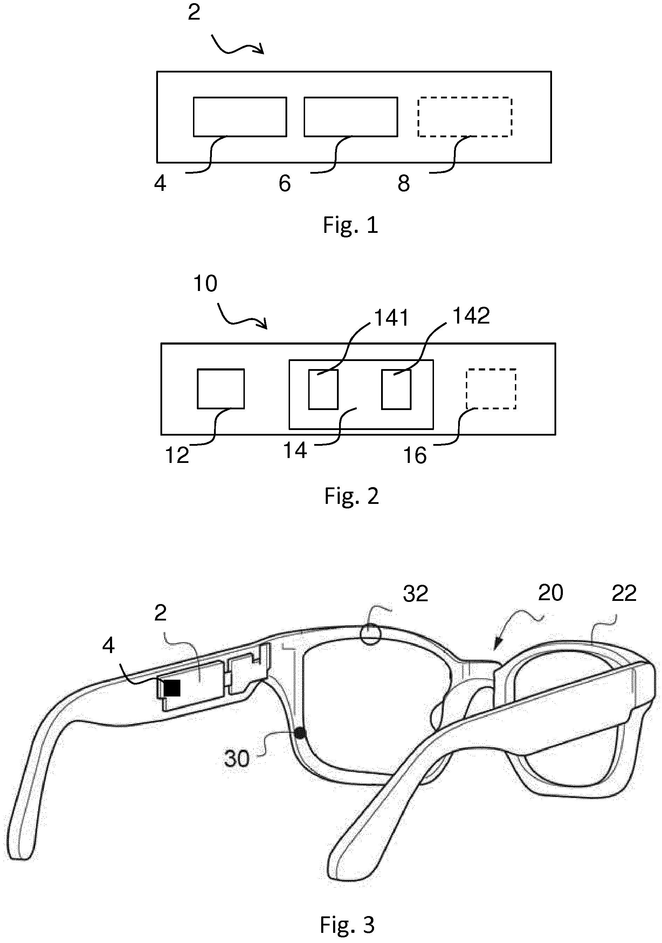

As represented on figure 1, the invention relates to a heart rate sensing module 2. The heart rate sensing module is configured to be fixed on a wearable device, the wearable device being intended to be worn by a user.

The wearable device is a head mounted device such as a spectacle frame, as represented on figure 2, or a pair of shoes or a watch.

The heart rate sensing module 2 comprises at least a contactless sensor 4 and a communication entity 6.

The contactless sensor 4 is adapted to sense at least one parameter indicative of the distance between at least a reference point of the skin of the user and a reference point of the module, in particular of the contactless sensor, when the module is fixed to the wearable device when worn by the user in such a manner that the contactless sensor is distant from the skin of the user.

When the heart rate sensing module is fixed on the wearable device and said wearable device is worn by the user the distance between the contactless sensor, in particular the at least one reference point of the contactless sensor and the skin of the user, in particular the reference point of the of the skin of the use, is greater than or equal to 0.1 mm, for example greater than or equal to 1 mm and smaller than or equal to 1 cm, for example smaller than or equal to 7 mm.

So as to provide more accurate heart rate sensing, the heart rate sensing module may be configured to that when fixed on the wearable device and said wearable device worn by the user, the contactless sensor faces an artery or vein of the user.

The contactless sensor is a capacitive sensor. The capacitance measured by the capacitive sensor may be used as an indication of the distance between the sensor and the skin of the user.

Typically, the electrode of the capacitive sensor can be printed on a circuit board fixed onto a wearable device or can be mounted directly into the wearable device or printed with conductive ink directly on the wearable device. The electrode may be flexible and the sensor may be embedded into clothes, for example using conductive wires like sliver nanowires embedded into standard clothes.

When the electrode of the capacitive sensor is approached by a distant object, like a finger or human skin, the capacitance of the circuit changes. The formula to determine capacitance is as follow :$C = \left(ε0∗εr\right) ∗ S / e ,$with
ε0 is the air dielectric constant,
εr is the relative dielectric constant of the material between skin and electrode,
e is the distance between the electrode and the distant object,
S is the surface of the electrodes

Blood when flowing in the vein and arteria induces a vein and arteria diameter variation and induces a small skin variation. Such skin variation can be detected by a capacitance measurement between the sensor and the skin of the user facing the sensor at a distance greater than or equal to 1 mm and smaller than or equal to 1cm.

Standard electronic chips are available to measure the capacitance variation induced by the distance variation between the sensor and the skin due to blood flow.

The contactless sensor may also be a magnetic sensor measuring the variation of magnetic field between two magnetic elements, one on the wearable device and one on the skin of the user, for example a magnet is stuck on the user's skin. In this case the magnetic sensor is on the wearable device and measure the magnetic field variation due to skin movement when blood flow.

The communication entity 6 of the heart rate sensing module 2 is adapted to communicate data indicative of the at least one parameter sensed by the at least one sensor to a heart rate determining component.

The data indicative of the at least one parameter sensed by the at least one sensor may be filtered to improve the accuracy, for example using a specific software and by selecting only the bandwidth of the heartbeat. This could be done using Fourrier transform or other frequency filtering methods.

As illustrated on figure 1, the heart rate sensing module may comprise a memory 8 to store the data prior to sending them to a heart rate determining component.

The heart rate sensing module may further comprise a power source, such as a battery, or may be electrically connected to a power source of a heart rate determining component or a power source comprised in the wearable device.

The invention further relates to a heart rate determining component for determining the heart rate of a wearer of a wearable sensing device according to the invention.

As illustrated on figure 2, the heart rate determining component 10 according to the invention comprises a communication unit 12 and a heart rate unit 14.

The communication unit 12 is configured to receive data indicative of the at least one parameter sensed by the at least one sensor of a heart rate sensing module according to the invention.

The communication unit 12 communicates with the communication entity 6 of a heart rate sensing module of the invention. Such communication may be wireless or both communication units may be linked electrically. Wireless communication can be done through different communication and protocols, like Bluetooth, Zigbee, WiFi or others.

The heart rate unit comprises at least a memory 141 and a processor 142. The memory 141 stores computer executable instructions. In addition to the computer executable instructions, the memory may store data such as at least part of the data received by the communication unit 12 or part of the data determined by the processor when executing the computer executable instructions stored in the memory.

The processor 142 is configured to execute the computer executable instructions stored in the memory 141. The stored computer executable instructions comprise instructions for determining heart rate data indicative of the heart rate of the user based on the received data.

According to an embodiment, the computer executable instructions further comprises instructions for determining heart rate data based on the variation over time of the distance between the at least a reference point of the skin of the user and a reference point of the module when the module is fixed to the wearable device worn by the user.

As illustrated on figure 2, the heart rate determining component 10 may further comprise an information generating unit 16 configured to generate information based on the heart rate data.

The information may comprise the information comprises recommendation data including a change of activity recommendation and/or an alert indicative of the user's heart state and/or an access to a service offer.

The communication unit 12 may be further configured to send heart rate related data to a distant entity, for example a wearable computer device and/or a personal computer device, associated to the user of the heart rate determining component. The heart rate related data may be information generated by the information generating unit 16 or data indicative of the heart rate of the user to be processed by the distant entity.

The heart rate determining component may further comprise a power source, such as a battery, or may be electrically connected to a power source of a heart rate sensing module or a power source comprised in the wearable device.

The invention further relates to a wearable device comprising a heart rate module according to the invention. The wearable device comprises a heart rate determining component arranged to receive data from the heart rate module.

Figure 3 illustrates an example of a wearable device, for example a head-mounted device 20, comprising a heart rate sensing module 2. The wearable device further comprises different type of sensors 30, 32. The head mounted device 20 comprises a spectacle frame 12 and the sensors and communication unit are mounted on the spectacle frame 12.

Although the invention is not limited to such type of wearable device, head-mounted device, appear to be particularly advantageous, in particular head mounted devices comprising a spectacle frame.

Indeed, such type of head mounted device is more likely to positioned over a long period of time facing a skin of the wearer, in particular facing the posterior auricular artery or vein.

The sensors 30 and 32 are configured to sense at least one feature indicative of the relative position of the head mounted device and at least one reference point of the head of the user of the head mounted device.

Advantageously, having an indication of the relative position of the head mounted device and the head of the user can be used to increase the accuracy of the measurements of the heart rate using the heart rate sensing module of the invention. Indeed, the heart rate sensing module senses data indicative of the distance between the sensor and the skin of the user. Thus, having an indication of the relative position of the sensor and the skin over time may be correlated to the data provided by the heart rate sensing module to increase the accuracy.

At least one of the additional sensors is a time of flight sensor configured to sense the distance between the head mounted device and the user of the head mounted device, for example the user's head. Such sensor may be placed on the frame in such position that when the head mounted device is worn by the user the sensor points to the face of the wearer. The sensor typically uses time of flight (TOF) technology and is arranged to provide data indicative of the distance to the head depending of the position of the head mounted device.

According to an embodiment of the invention, at least one of the additional sensor is a proximity sensor based on infrared reflectance on the user skin so as to sense the proximity between the head mounted device and the user of the head mounted device, for example the user's head.

Such infrared reflectance sensor may advantageously be placed on the nose bridge of the frame of the head mounted device and oriented toward the nose so as to provide data indicative of the distance to the skin of the user, for example of the sellion of the user. Advantageously, when the head mounted device is worn by the user, the nose bridge sensor is very close to the skin of the user, and if the position of the head mounted device changes this distance increases and can be easily detected.

The time of flight and the infrared reflectance sensors may require training to detect distance during the different use of the head mounted device, and detection of the different positions occur after statistical analysis of the data provided by the sensors.

According to a further embodiment of the invention, one of the additional sensors is an accelerometer and/or gyroscope configured to sense the orientation of the head mounted device.

Typically, in a first reference position, the head mounted device is almost horizontal, and in other position the head mounted device may be oriented upward or downward, this inclination can be measured by 3 or 6 axis sensors commonly used in mobile phone. Typically, a 3 axis accelerometer can provide data concerning the horizontality of the head mounted device and a 3 axis gyroscope can provide data concerning rotation of the head mounted device. Also the movements of the head mounted device from one position to another can be detected, and differentiated from head movement to be sure to detect a head mounted device relative movement. In some case only accelerometer can be used, to save power.

The different type of sensors provide features indicative that need to be processed so as to determine information indicative of the relative position of the head mounted device and the user of the head mounted device.

Although not represented, the head mounted device may further comprise a power source, for example a battery and/or other electronics.

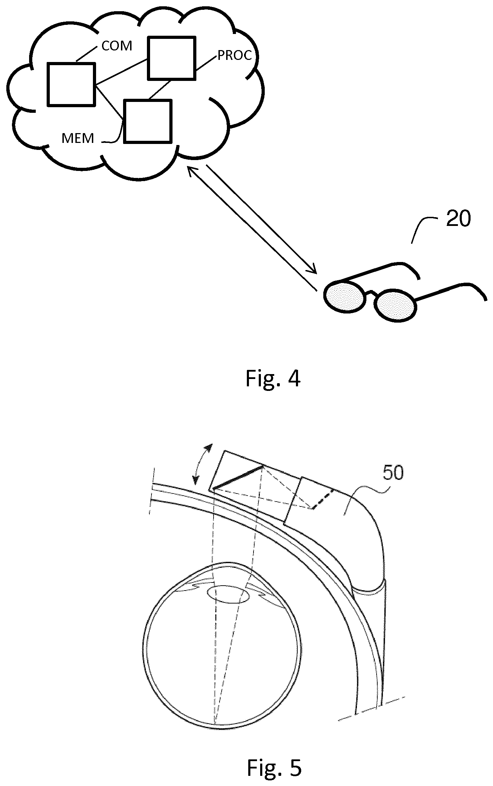

According to an embodiment of the invention, illustrated on figure 4, the head mounted device communicates with a distant entity that comprises a heart rate determining component. Communication can be done through different communication devices and protocols, like Bluetooth, Zigbee, WiFi or others.

For example, the communication unit is configured to communicate with the distance entity either to store the measured features in a memory MEM or to provide information relating to the heart rate of the user.

Typically, the distant entity comprises a heart rate determining component according to the invention.

The distance entity can include different computing objects such as personal digital assistants, audio/video devices, mobile phones, MPEG-1 Audio Layer 3 (MP3) players, personal computers, laptops, tablets, bluetooth headset, watch, wristband, etc...

Each computing object and the head mounted device can communicate with one or more other by way of a communication network, either directly or indirectly. Even though illustrated as a single element in figure 4, network can include other computing objects and computing devices that provide services to the system of figure 4, and/or can represent multiple interconnected networks, which are not shown.

In a network environment in which the communications network/bus can be the Internet, the computing objects can be Web servers, file servers, media servers, etc. with which the client computing objects or devices communicate via any of a number of known protocols, such as the hypertext transfer protocol (HTTP).

In an alternative embodiment, the heart rate determining component may be embodied in the wearable device and electrically connected to the heart rate sensing module.

According to an embodiment of the invention, the data received by the heart rate determining component from the heart rate sensing module may be stored over time and the information is indicative of the evolution over time of the heart rate of the user.

Furthermore the information generating unit may be configured to generate information using statistical analysis of the data received from the head mounted device and stored over time.

Statistics involves the collection, organization, analysis, interpretation, and/or presentation of measured/collected information. With advances in technology, more extensive and complex computing allows massive amounts of data to be collected, stored and/or processed. Further, methods for evaluating the data are numerous.

Statistical analysis can be employed to process and/or evaluate data sensed.

As represented on figure 4, the head mounted device according to the invention may comprise a virtual image display device 50, preferably allowing the wearer to see both the virtual image and the real world through it. The virtual image display device is able to display graphical images, and an electronic driving system (memory + processor) sends to the virtual display image the image to display. For example the heart rate over time may be displayed or an alert may be displayed if a heart or health defect is detected.

The invention has been described above with the aid of embodiments without limitation of the general inventive concept.

Many further modifications and variations will suggest themselves to those skilled in the art upon making reference to the foregoing illustrative embodiments, which are given by way of example only and which are not intended to limit the scope of the invention, that being determined solely by the appended claims.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used. Any reference signs in the claims should not be construed as limiting the scope of the invention.

## Claims

1. A head mounted device comprising a heart rate sensing module (2) configured to be fixed on the head mounted device intended to be worn by a user, the module comprising:
- a heart rate determining component (10) for determining the heart rate of a wearer of the head mounted device, the heart rate determining component comprising:
- a communication unit (12) configured to receive data indicative of the at least one parameter sensed by the at least one sensors (4) of the heart rate sensing module (2), and
- a heart rate unit (14) comprising at least
- a memory (141) configured to store computer executable instructions; and
- a processor (142) for executing the computer executable instructions,
wherein the computer executable instructions comprise instructions for determining heart rate data indicative of the heart rate of the user based on the received data,
- wherein the at least one sensor is at least one contactless sensor (4) adapted to sense at least one parameter indicative of a distance between at least a reference point of the skin of the user and a reference point of the module when the module is fixed to the head mounted device when worn by the user in such a manner that the contactless sensor is distant from the skin of the user,
- at least one communication entity (6) adapted to communicate data indicative of the at least one parameter sensed by the at least one sensor to the communication unit of the heart rate determining component,
wherein the head mounted device further comprises different types of sensors (30, 32) configured to determine information indicative of the relative position of the head mounted device to the user of the head mounted device,
wherein the relative position corresponds to the distance between the sensor and the skin of the user,
wherein the heart rate sensing module is configured so that when fixed on the head mounted device and said head mounted device when worn by the user, the distance between the contactless sensor (4) and the skin of the user is greater than or equal to 0.1 mm and smaller than or equal to 5 mm,
wherein the different type of sensors (30, 32) are configured to sense at least one feature indicative of the relative position of the head mounted device and at least one reference point of the head of the user of the head mounted device,
wherein the device is configured to correlate an indication of the relative position of the contactless sensor (4) and the skin over time is correlated to the data provided by the heart rate sensing module to increase the accuracy of the measurements of the heart rate using the heart rate sensing module,
wherein at least one of the different type of sensors is a time of flight sensor configured to sense the distance between the head mounted device and the user of the head mounted device, for example the user's head,
wherein the contactless sensor is a capacitive sensor and the at least one parameter relates to a capacitance measured by the capacitive sensor,

2. The head mounted device according to claim 1, wherein the head mounted device is a spectacle frame.

3. The head mounted device according to any of the preceding claims, wherein the heart rate sensing module is fixed on the head mounted device so that when the heart rate sensing module is fixed on the head mounted device and the head mounted device is worn by the user, the contactless sensor (4) faces an artery or vein of the user.

4. The head mounted device according to any of claims 1 to 3, wherein the contactless sensor (4) is a magnetic sensor.

5. The head mounted device according to any of the preceding claims, wherein the computer executable instructions of the heart rate determining component further comprises instructions for determining heart rate data based on the variation over time of the distance between the at least a reference point of the skin of the user and a reference point of the module when the module is fixed to the head mounted device worn by the user.

6. The head mounted device according to any of the preceding claims, wherein the heart determining component further comprises an information generating unit configured to generate information based on the heart rate data.

7. The head mounted device according to the preceding claim, wherein the information comprises recommendation data including a change of activity recommendation and/or an alert indicative of the user's heart state and/or an access to a service offer.

8. The head mounted device according to any of the preceding claims, wherein the communication unit (12) is configured to send heart rate related data to a distant entity, for example a wearable computer device and/or a personal computer device, associated to the user of the heart rate determining component.

## Patentansprüche

1. Am Kopf aufgesetzte Vorrichtung, umfassend ein Herzfrequenzerfassungsmodul (2) , das dazu konfiguriert ist, auf der am Kopf aufgesetzten Vorrichtung, die von einem Benutzer getragen werden soll, befestigt zu werden, wobei das Modul umfasst:
- eine Herzfrequenzbestimmungskomponente (10) zum Bestimmen der Herzfrequenz eines Trägers der am Kopf aufgesetzten Vorrichtung, wobei die Herzfrequenzbestimmungskomponente Folgendes umfasst:
- eine Kommunikationseinheit (12), die dazu konfiguriert ist, Daten zu empfangen, die den mindestens einen Parameter angeben, der durch den mindestens einen Sensor (4) des Herzfrequenzerfassungsmoduls (2) erfasst wird, und
- eine Herzfrequenzeinheit (14), die zumindest umfasst
- einen Speicher (141), der zum Speichern von computerausführbaren Anweisungen konfiguriert ist; und
- einen Prozessor (142) zum Ausführen der computerausführbaren Anweisungen,
wobei die computerausführbaren Anweisungen Anweisungen zum Bestimmen von Herzfrequenzdaten, die die Herzfrequenz des Benutzers angeben, basierend auf den empfangenen Daten umfassen,
- wobei der mindestens eine Sensor mindestens ein berührungsloser Sensor (4) ist, der dazu ausgelegt ist, mindestens einen Parameter zu erfassen, der einen Abstand zwischen mindestens einem Referenzpunkt der Haut des Benutzers und einem Referenzpunkt des Moduls angibt, wenn das Modul an der am Kopf aufgesetzten Vorrichtung befestigt ist, wenn sie von dem Benutzer auf eine solche Weise getragen wird, dass der berührungslose Sensor von der Haut des Benutzers entfernt ist,
- mindestens eine Kommunikationsentität (6), die dazu ausgelegt ist, Daten, die den mindestens einen durch den mindestens einen Sensor erfassten Parameter angeben, an die Kommunikationseinheit der Herzfrequenzbestimmungskomponente zu kommunizieren, wobei die am Kopf aufgesetzte Vorrichtung ferner verschiedene Typen von Sensoren (30, 32) umfasst, die dazu konfiguriert sind, Informationen zu bestimmen, die die relative Position der am Kopf aufgesetzten Vorrichtung zum Benutzer der am Kopf aufgesetzten Vorrichtung angeben,
wobei die relative Position dem Abstand zwischen dem Sensor und der Haut des Benutzers entspricht,
wobei das Herzfrequenzerfassungsmodul so konfiguriert ist, dass dann, wenn es auf der am Kopf aufgesetzten Vorrichtung befestigt ist, und wenn die am Kopf aufgesetzte Vorrichtung vom Benutzer getragen wird, der Abstand zwischen dem berührungslosen Sensor (4) und der Haut des Benutzers größer oder gleich 0,1 mm und kleiner oder gleich 5 mm ist,
wobei die unterschiedlichen Typen von Sensoren (30, 32) dazu konfiguriert sind, mindestens ein Merkmal, das die relative Position der am Kopf aufgesetzten Vorrichtung angibt, und mindestens einen Referenzpunkt des Kopfes des Benutzers der am Kopf aufgesetzten Vorrichtung zu erfassen,
wobei die Vorrichtung dazu konfiguriert ist, eine Angabe der relativen Position des berührungslosen Sensors (4) und der Haut über den Zeitverlauf zu korrelieren, was mit den Daten korreliert wird, die durch das Herzfrequenzerfassungsmodul bereitgestellt werden, um die Genauigkeit der Messungen der Herzfrequenz unter Verwendung des Herzfrequenzerfassungsmoduls zu erhöhen, wobei mindestens einer der unterschiedlichen Typen von Sensoren ein Flugzeitsensor ist, der dazu konfiguriert ist, den Abstand zwischen der am Kopf aufgesetzten Vorrichtung und dem Benutzer der am Kopf aufgesetzten Vorrichtung, zum Beispiel dem Kopf des Benutzers, zu erfassen,
wobei der berührungslose Sensor ein kapazitiver Sensor ist und sich der mindestens eine Parameter auf eine durch den kapazitiven Sensor gemessene Kapazität bezieht.

2. Am Kopf aufgesetzte Vorrichtung nach Anspruch 1, wobei die am Kopf aufgesetzte Vorrichtung ein Brillengestell ist.

3. Am Kopf aufgesetzte Vorrichtung nach einem der vorhergehenden Ansprüche, wobei das Herzfrequenzerfassungsmodul auf der am Kopf aufgesetzten Vorrichtung befestigt ist, so dass, wenn das Herzfrequenzerfassungsmodul auf der am Kopf aufgesetzten Vorrichtung befestigt ist und die am Kopf aufgesetzte Vorrichtung vom Benutzer getragen wird, der berührungslose Sensor (4) einer Arterie oder Vene des Benutzers zugewandt ist.

4. Am Kopf aufgesetzte Vorrichtung nach einem der Ansprüche 1 bis 3, wobei der berührungslose Sensor (4) ein magnetischer Sensor ist.

5. Am Kopf aufgesetzte Vorrichtung nach einem der vorhergehenden Ansprüche, wobei die computerausführbaren Anweisungen der Herzfrequenzbestimmungskomponente ferner Anweisungen zum Bestimmen von Herzfrequenzdaten basierend auf der zeitlichen Variation des Abstands zwischen dem mindestens einen Referenzpunkt der Haut des Benutzers und einem Referenzpunkt des Moduls umfassen, wenn das Modul an der vom Benutzer getragenen am Kopf aufgesetzten Vorrichtung befestigt ist.

6. Am Kopf aufgesetzte Vorrichtung nach einem der vorhergehenden Ansprüche, wobei die Herzbestimmungskomponente ferner eine Informationserzeugungseinheit umfasst, die dazu konfiguriert ist, Informationen basierend auf den Herzfrequenzdaten zu erzeugen.

7. Am Kopf aufgesetzte Vorrichtung nach dem vorhergehenden Anspruch, wobei die Informationen Empfehlungsdaten umfassen, die eine Empfehlung zur Änderung der Aktivität und/oder eine Warnung beinhalten, die den Herzzustand des Benutzers und/oder einen Zugriff auf ein Serviceangebot anzeigt.

8. Am Kopf aufgesetzte Vorrichtung nach einem der vorhergehenden Ansprüche, wobei die Kommunikationseinheit (12) dazu konfiguriert ist, herzfrequenzbezogene Daten an eine entfernte Entität, zum Beispiel eine tragbare Computervorrichtung und/oder eine Personal-Computer-Vorrichtung, die dem Benutzer der Herzfrequenzbestimmungskomponente zugeordnet ist, zu senden.

## Revendications

1. Dispositif monté sur la tête comprenant un module de détection de fréquence cardiaque (2) configuré pour être fixé sur le dispositif monté sur la tête destiné à être porté par un utilisateur, le module comprenant :
- un composant de détermination de fréquence cardiaque (10) pour déterminer la fréquence cardiaque d'un porteur du dispositif monté sur la tête, le composant de détermination de fréquence cardiaque comprenant :
- une unité de communication (12) configurée pour recevoir des données représentatives de l'au moins un paramètre détecté par l'au moins un capteur (4) du module de détection de fréquence cardiaque (2), et
- une unité de fréquence cardiaque (14) comprenant au moins
- une mémoire (141) configurée pour stocker des instructions exécutables par ordinateur, et
- un processeur (142) pour exécuter les instructions exécutables par ordinateur,
les instructions exécutables par ordinateur comprenant des instructions pour déterminer des données de fréquence cardiaque indicatives de la fréquence cardiaque de l'utilisateur sur la base des données reçues,
- l'au moins un capteur étant au moins un capteur sans contact (4) adapté pour détecter au moins un paramètre indicatif d'une distance comprise entre au moins un point de référence de la peau de l'utilisateur et un point de référence du module lorsque le module est fixé sur le dispositif monté sur la tête lorsqu'il est porté par l'utilisateur de telle façon que le capteur sans contact est distant de la peau de l'utilisateur,
- au moins une entité de communication (6) adaptée pour communiquer des données indicatives de l'au moins un paramètre détecté par l'au moins un capteur à l'unité de communication du composant de détermination de fréquence cardiaque,
le dispositif monté sur la tête comprenant en outre différents types de capteurs (30, 32) configurés pour déterminer des informations indicatives de la position relative du dispositif monté sur la tête par rapport à l'utilisateur du dispositif monté sur la tête,
la position relative correspondant à la distance entre le capteur et la peau de l'utilisateur,
le module de détection de fréquence cardiaque étant configuré pour que, lorsqu'il est fixé sur le dispositif monté sur la tête et ledit dispositif monté sur la tête lorsqu'il est porté par l'utilisateur, la distance entre le capteur sans contact (4) et la peau de l'utilisateur est supérieure ou égale à 0,1 mm et inférieure ou égale à 5 mm,
les différents types de capteurs (30, 32) étant configurés pour détecter au moins une caractéristique indicative de la position relative du dispositif monté sur la tête et au moins un point de référence de la tête de l'utilisateur du dispositif monté sur la tête,
le dispositif étant configuré pour corréler, une indication de la position relative du capteur sans contact (4) et de la peau, au cours du temps, avec les données fournies par le module de détection de fréquence cardiaque pour augmenter la précision des mesures de la fréquence cardiaque à l'aide du module de détection de fréquence cardiaque,
au moins un des différents types de capteurs étant un capteur de temps de vol configuré pour détecter la distance entre le dispositif monté sur la tête et l'utilisateur du dispositif monté sur la tête, par exemple la tête de l'utilisateur,
le capteur sans contact étant un capteur capacitif et l'au moins un paramètre étant relatif à une capacité mesurée par le capteur capacitif,

2. Dispositif monté sur la tête selon la revendication 1, le dispositif monté sur la tête étant une monture de lunettes.

3. Dispositif monté sur la tête selon l'une quelconque des revendications précédentes, le module de détection de fréquence cardiaque étant fixé sur le dispositif monté sur la tête de sorte que lorsque le module de détection de fréquence cardiaque est fixé sur le dispositif monté sur la tête et que le dispositif monté sur la tête est porté par l'utilisateur, le capteur sans contact (4) fait face à une artère ou à une veine de l'utilisateur.

4. Dispositif monté sur la tête selon l'une quelconque des revendications 1 à 3, le capteur sans contact (4) étant un capteur magnétique.

5. Dispositif monté sur la tête selon l'une quelconque des revendications précédentes, les instructions exécutables par ordinateur du composant de détermination de fréquence cardiaque comprenant en outre des instructions pour déterminer des données de fréquence cardiaque sur la base de la variation dans le temps de la distance entre l'au moins un point de référence de la peau de l'utilisateur et un point de référence du module lorsque le module est fixé au dispositif monté sur la tête porté par l'utilisateur.

6. Dispositif monté sur la tête selon l'une quelconque des revendications précédentes, le composant de détermination de fréquence cardiaque comprenant en outre une unité de génération d'informations configurée pour générer des informations sur la base des données de fréquence cardiaque.

7. Dispositif monté sur la tête selon la revendication précédente, les informations comprenant des données de recommandation comprenant une recommandation de changement d'activité et/ou une alerte indiquant l'état cardiaque de l'utilisateur et/ou un accès à une offre de service.

8. Dispositif monté sur la tête selon l'une quelconque des revendications précédentes, l'unité de communication (12) étant configurée pour envoyer des données relatives à la fréquence cardiaque à une entité distante, par exemple un dispositif informatique portable et/ou un dispositif informatique personnel, associé à l'utilisateur du composant de détermination de fréquence cardiaque.
